# EUROPEAN PATENT APPLICATION

(11) **EP 3 721 783 A1**
(43) Date of publication of application: **14.10.2020**
(21) Application number: 19382263.2
(22) Date of filing: 09.04.2019
(51) Int. Cl.: A61B 1/00, A61B 1/005, A61B 1/018

(54) **VIDEOSCOPE**

(71) Applicant: Visual Oxy, S.L., Málaga (ES)
(72) Inventor: MONTERO GONZÁLEZ, Emilio, 29002 Málaga (ES)
(74) Representative: Elzaburu S.L.P.

(57) **Abstract**

Videoscope with a grip (100) and a tube (200) with mini-camera where the grip comprises two articulated parts by means of a hinge, and a connection piece housed within them. Thus, the grip may be disposed in an open position and a closed position, creating an accessible inner cavity (107) in the open position of the grip. The connection piece comprises: mechanical connection means with a proximal end of the mini-camera tube; electric power connection means, configured to transmit electric power to the mini-camera, and; electronic connection means with the mini-camera tube, configured for image data transmission. Preferably, the grip comprises a protector shield (110) to avoid accidental pricking with needles inserted in the inlet of the working channel, and the tube with the mini-camera comprises a flexible piece in its distal part, manufactured in a single piece.

## Description

### Purpose of the invention

The present invention has the purpose of a videoscope or fiberscope, with a modular configuration, which allows a considerable saving in costs with respect to other conventional fiberscopes or videoscopes. In addition to introducing a flexible tip system formed by joined links which give greater lateral and longitudinal stability to the flexible tip in its vertical movements upward or downward and horizontal movements right or left. It also incorporates a defence or protection system formed by a plate in the proximal inlet of the working channel of the videoscope or fiberscope. It further incorporates a novel fastening system between the cables that transmit the movement to the flexible tip and the actuator or handle that directs it called double fastening with pulley.

The videoscope object of the present invention has special application in the field of the industry dedicated to the design, manufacturing and marketing of surgical instruments, in particular instruments designed for the internal observation of organs and the operation and/or sample taking from the inside thereof.

### Background of the invention and technical problem to resolve

A videoscope or fiberscope is a device which is used for the viewing/monitoring of the human body and, in some types of videoscopes, also for the work inside the human body whilst it is viewed.

The videoscope is mainly composed of two parts, in first place, by a grip/body which is held by the doctor's hand which is solidly joined to a tube that is flexible in its distal part and carries therein a camera at its distal end, and a second piece, which is a monitor that can either be external and whereto it is connected by means of a cable, or may be a portable monitor that can be coupled to the grip by its proximal end.

Each videoscope is designed and specialized for different medical branches or specializations, thus distinguishing between: bronchoscope, gastroscope, colonoscope, laryngoscope, etc.

In addition to this classification, it can be distinguished according to its reuse as reusable or single-use fiberscope.

There are several common characteristics in the grip/body of all conventional fiberscopes or videoscopes existing on the market such as the handle or actuator which rotates upward or downward to control by means of cables whereto it is joined the movement of the distal part of the tip of the tube which is introduced in the human body and which incorporates a mini-camera. The movements of the flexible tip may be only in vertical direction upward or downward, or also include the movement in horizontal direction right or left.

There are some characteristics that differentiate all the fiberscopes or videoscopes, which are on occasions an extra of functionality, such as the possibility that characteristics are included in part of the grip/body, such as the coupling of a screen or monitor to make the device more portable/independent. The large majority of the fiberscopes or videoscopes, instead of having a coupled monitor, use a cable connection directly to an external fixed monitor.

Another characteristic that differentiates between them is that some operate with rechargeable or non-rechargeable batteries, whilst other fiberscopes or videoscopes need to be connected to an external power source.

Likewise, it should be highlighted that, in many videoscopes/fiberscopes, it is possible to find a connection/orifice in the body or grip that is proximally guided from said inlet in the grip to the distal end of the tube which enters in the patient's body, finishing its exit beside the position of the mini-camera, also in the distal part or end of the tube. This connection/orifice is called working channel. It is used to perform different functions whilst it is displaying an area of the anatomy, functions such as applying the necessary medicines, introducing oxygen, aspiration of secretions, introducing a type of catheter, measurement, biopsy means, needles, etc. Furthermore, the fiberscopes or videoscopes are differentiated according to this characteristic among those that do not have a working channel and those that do have a working channel, and within the first, those wherein the working channel is of a small diameter to introduce oxygen or for aspiration, and those wherein the diameter of the working channel is large for the introduction of needles and to perform biopsies. In the case of the introduction of needles and biopsy means through the working channel, the danger lies in that in current videoscopes and fiberscopes, the doctor's hand is not protected from the inlet of the working channel, so that on inserting the needle through the working channel the doctor's hand runs the risk of being pricked with the needle and the doctor getting infected.

Aside from all the optional and differentiating characteristics described above, fiberscopes or videoscopes also have other important characteristics that differentiate between them, such as the length of the tube that integrates the mini-camera, the external diameter of said tube, the rotation capacity of the distal and flexible part of said tube (90 degrees or 180 degrees, in a single plane, which would be the vertical movement upward or downward (2 axes), or in two perpendicular planes which would be the vertical movement plus the horizontal movement right or left (4 axes), etc.); thus, there are fiberscopes/videoscopes whose channels have, in their distalmost part, a larger or smaller rotation capacity and a rotation capacity in one plane or two planes.

Another difference between videoscopes is the resolution of the optical quality of the mini-camera: there are 320x240, others are High Definition and of greatest resolution (called Full HD). There are many types of optical resolution depending on clinical requirements.

Of all the characteristics described above, it is gathered that there are very many different types of fiberscope or videoscope, according to the necessary characteristics for each type of patient and medical specialization.

In the state of the art, a videoscope/fiberscope is a compact device where the grip always goes together with its actuator or handle that controls the movement of the flexible distal part of the tube by means of cables whereto it is fastened, the connections to the external monitor, or the connections for coupling the portable monitor, the tube which integrates the mini-camera and the working channel. Every time it is used in a patient, either the entire fiberscope/videoscope is reused as a whole (with the requirement and cost it entails of sterilization and re-sterilization and the infection problems related to re-sterilization), or it is disposed of and the entire device is discarded in the disposables bin (which entails a great cost to eliminate the entire device and the need to purchase another complete device for the next patient).

Both the sterilization and the problems of breakage of the device and infections due to re-sterilization, and the great cost of disposing of or discarding the entire device in the single-use models entails a great financial impediment for customers when acquiring products of critical need, vital for operating theatre work, ICU, accident and emergency, since not all hospitals have the same economic level in different countries, not even in the same country.

Another of the drawbacks observed in current fiberscopes is in the distal part of the tube which is flexible due to the articulation of the distal end of the mini-camera tube, which allows that said mini-camera can be oriented in different directions in one or two planes during the display by means of the fiberscope/videoscope.

This articulated part in the distal part of the tube has the capacity to move upward and downward due to the transmission of the movement of the cables together with the handle or actuator, as it moves with the actuator or, as it moves with the actuator or handle, and which will direct the vision of the mini-camera to the area the doctor wants.

This articulated part is manufactured in several mechanical ways, but all of them have several faults: one of them is a fault of instability and misalignment both in lateral and longitudinal direction; another fault of that articulated part is of creating pinching with the plastic cover that covers it, creating sharp edges on dismantling the mechanical components that form it, creating folds in the plastic which surrounds that articulated part, and all these problems come from that longitudinal misalignment or longitudinal stretching and of the lateral misalignment of the articulated part's components. The articulated end part of all the videoscopes/fiberscopes has to fulfil functions, such as that of being able to rotate from one side to another and also the function of being able to hold therein the electric or electronic cables that carry the image from the mini-camera to the grip and to the monitor and also carry therein the electricity of the grip to the mini-camera to be able to work, and finally, the articulated part has the function of carrying therein the two or four tensioners or movement cables that come from the handle or actuator and which join to the distalmost part of the tube that integrates the mini-camera, so that on pulling them the articulated distal part of the tube rotates towards one side or the other.

These two or four tensioners or movement cables and the two or three electric or electronic electricity and image transmission cables have to go through independent channels, and not mixing each one of them within the articulated part of the flexible tube. Otherwise, the upward and downward or lateral movement cannot be performed in controlled manner and the electric or electronic cables would be affected by the continuous friction with said tensioners/movement cables, and may break and short-circuit or lose the mini-camera's vision.

The pinching, sharp edges or folds when the tube bends or folds are hazardous for patients as they may cause erosions, chaffing, bleeding, trauma, when the distalmost part of the tube bends, in addition to the videoscope losing efficacy in its movement control and accuracy in vision.

This lateral and longitudinal instability and misalignment of the articulated flexible part (in the distalmost end of the tube) is due to the inner pieces or components of the articulated part being released. The method currently used by videoscopes/fiberscopes to create that bending movement is of independent pieces that are coupled one to another and rotate, but this coupling or joining between the independent pieces does not have a stable longitudinal and lateral joint. Those internal mechanical pieces or components are easily released with lateral traction, longitudinal traction or stretching and with continued use. Furthermore, these coupled independent pieces are manufactured in metal which rusts on being put in sterilization and re-sterilization liquids.

Due to this, not only can the fiberscope not continue to be used as its movement is incontrollable, but hazardous problems may appear for the patient's safety due to pinching in the plastics that surround the articulated part and the sharp edges that laterally protrude due to the detachment of the units or independent components, these problems are internal haemorrhages due to the wounds produced by the pinching, folds or sharp edges.

Another problem all videoscopes/fiberscopes have is the manner in which the handle or actuator is fastened to the tensioning cables that carry the movement to the flexible tip of the tube. This takes us to the greatest problem existing in videoscopes/fiberscopes, which is that the tensioning cables come off from the handle or actuator and the device can no longer be used or controlled.

### Description of the invention

With the aim of resolving the aforementioned drawbacks, the present invention relates to a videoscope or fiberscope with improved characteristics with respect to conventional videoscopes or fiberscopes.

The videoscope/fiberscope object of the present invention includes a grip and a tube equipped with a mini-camera at a distal end of the tube.

In a novel manner, the grip comprises two parts, articulated together by means of a hinge situated at a first end of the grip.

The grip comprises closing means of the two parts (preferably at a second end of the grip), so that the grip is susceptible of being disposed in an open position and a closed position.

The grip comprises an accessible inner cavity in the open position of the grip. The grip cavity is configured to house a connection piece (which is preferably covered by the two parts of the grip when the grip is in its closed position.

The connection piece comprises:
- mechanical connection means with a proximal end of the mini-camera tube, configured to stabilize the proximal end of the tube in the grip cavity;
- electric power connection means, configured to transmit electric power to the mini-camera, and;
- electronic connection means with the mini-camera tube, configured for image data transmission.

Preferably, the electronic connection means are connected to a data outlet port, for the connection of a data cable and/or a removable data storage unit to the videoscope grip.

Alternatively or additionally, the electronic connection means may be connected to an internal data storage unit.

According to one possible embodiment, the electric power connection means are connected to an internal battery situated in the videoscope grip.

Alternatively or additionally, the electric power connection means may be connected to a connection port of the videoscope grip for the connection of a power cable configured to be connected to an external power source.

Preferably, in conjunction with the previous characteristics, or independently from them, the videoscope tube comprises a working channel configured for the passage of a needle or biopsy means through the inside of said tube, and the grip comprises a proximal inlet of said working channel, said inlet being configured for the introduction of the needle or biopsy means. Advantageously, the grip comprises a protector shield between said proximal inlet and the gripping area of the grip for the doctor's hand. By means of this protector shield, it reduces or avoids the risk of pricking the hand of the person holding the grip due to using needles or biopsy means.

The distal part of the videoscope tube may also comprise a novel flexible tip, which replaces articulated tips (metal and multiple pieces and shafts) of conventional videoscopes.

The flexible tip for videoscope is made in a single piece and comprises a plurality of links joined together by means of at least one column. There are preferably at least two columns.

Said at least one (preferably at least two columns) column comprises/comprise a narrowing in a central section between every two links, thus allowing the bending of the tip in each narrowing area of the column.

Each link comprises a geometry with a plurality of curves or hollows, so that, on disposing links adjacent to other links, said curves or hollows generate axial channels of the entire structure of the flexible tip. These axial channels are independent when one looks at the entire piece from the front part. These channels may be at minimum 3 for the flexible tips that rotate in a single vertical plane, or be at minimum 5 channels for the flexible tips that rotate in two vertical and horizontal planes. The necessary step from 3 to 5 channels is due to requiring two further tensioning cables to achieve the other two extra horizontal movements.

Each channel is configured for the separate introduction of tensioning cables or movement cables for movement of the flexible tip and of electric and electronic cables for power supply of the mini-camera and for image data transmission.

As already introduced, preferably, the flexible tip comprises at least three channels (for the tips of vertical movement in a single plane): a central channel (of greater section) configured for the electric and electronic cables, and two lateral channels (or an upper channel and another lower one) configured for the introduction of the tensioning cables.

Preferably, the flexible tip comprises at least two columns which join the links together giving lateral and longitudinal stability to the entire flexible piece, and allowing the rotation between links in a single plane marked or directed by these two joining columns between links.

Also preferably, the flexible tip is manufactured by means of plastic injection.

Preferably, the flexible tip comprises three different types of links.

According to one possible embodiment, the links are grouped so that each link is preceded and/or followed by a link of a different type.

According to another possible embodiment, the links are grouped in threes, forming groups of three links of the same type and where each group is preceded and/or followed by a group of links of a different type.

Also according to another possible embodiment, the links are grouped in twos, forming groups of two links of the same type and where each group is preceded and/or followed by a group of links of a different type.

Preferably, in conjunction with the previous characteristics, or independently from them, the videoscope has a joining system between at least one tensioning cable and the handle or actuator, where said joining system comprises two fastenings and a pulley situated on a support.

The first fastening, situated in a first end area of the pulley, is configured to perform a pretensioning of the tensioning cables during assembly of the videoscope in the production chain; this pretensioning does not serve to withstand the force exerted on the handle, but just for the correct initial tensioning of the tensioning cables; after the first fastening there is the pulley, which is configured to bear/withstand the actuation with force of the handle; finally, after the pulley, there is the second fastening, situated in a second end area of the pulley, which is configured to fasten the distal end of the tensioning cable after exiting the pulley, which fastens so that the pulley cable so that said tensioning cable does not come off the pulley.

This double-fastening system with pulley allows, with the first fastening, an initial optimum tensioning of the tensioning cables in the device's production chain, then with the pulley it allows withstanding the greater force exerted by the doctor on the handle during its use, and the second fastening fastens the cables so that the pulley is not released.

Preferably, the joining system provides the joining of at least two tensioning cables with the handle or actuator. Therefore, the joining system provides that there are two pulleys situated on the support, and consequently the first two fastenings situated in respective first end areas of each pulley, and two second fastenings situated in respective second end areas of each pulley.

According to this innovative invention, on making each fiberscope or videoscope in separate modules, where on the one hand all the elements that do not come into contact with the patient (grip and connections) and on the other, all the elements that are in contact with the patient (tube, mini-camera), they manage to reduce the costs related to the recycling and acquisition of new complete fiberscopes/videoscopes, and also the sterilization or re-sterilization costs of the entire fiberscope, with the associated risks of element breakage.

Each medical specialization uses a specific videoscope model with specific characteristics.

According to the present invention, the elements which do not come into contact with the patient (which are also the most voluminous) are the grip, the electrical connections with the external monitor and the electrical connections with the portable monitor.

The elements that do come into contact with the patient are the tube that integrates the mini-camera, which is flexible in its distal part and the working channel.

Likewise, as regards the flexible tip, it is designed so that, in its manufacturing by means of plastic injection the flexible tip comes out with all its links of a complete piece in each opening and closing movement of the two faces of the mould in the pressing process in the injection.

Furthermore, the flexible tip forms therein all the necessary channels, among them the two independent channels for the two tensioning cables or movement cables of bending and also the independent tube for the electric and electronic cables of the mini-camera in the case of the flexible tip dedicated to rotation in a vertical plane; and the 5 independent channels of the flexible tip dedicated to rotation in the two vertical and horizonal planes.

The fact that the flexible tip can be manufactured so that it comes out of one complete piece provides a huge economic value, since it greatly cheapens its production as there is not many loose pieces that need to be manually assembled.

Not having this manual assembly in the manufacturing method of the flexible tip that is performed in current videoscopes greatly reduces the number of rejections in assembly as there is no handling of the pieces and greatly facilitates the production quality controls.

The fact that the flexible piece is one piece and is plastic instead of metal, as in other conventional videoscopes, facilitates the use within this videoscope inside magnetic resonance equipment.

Likewise, the fact that it is of a single piece formed by links joined by a column (preferably joined by a minimum of two columns) between links gives it robustness and eliminates longitudinal and lateral misalignments or decoupling, eliminating the pinching and sharp edges that create bleeding and wounds.

The two columns, ribs or pillars allow the bending in one direction between the links on reducing their thickness (in the narrowing), but keeps their thickness thicker in the rest of the section of the pillars to give robustness, eliminating the lateral and longitudinal displacement.

In the present invention, the rotation or turn between links of the flexible tip is not achieved in the form of two independent elements that rotate between one another mechanically joined by a bolt that joins the individual pieces as occurs in the current bending systems. In contrast, in the present invention, the "rotation axes" of the flexible tip is generated from a reduction in the thickness in a specific area in the two disc joining pillars.

The way in which the flexible tip is designed allows lengthening the useful life of the product whilst it is used in the same patient, since there is no possibility of releasing the structure that forms it, as it is composed of a single piece.

Likewise, by means of the modular model with grip made in two parts, it is possible to have all the different models of videoscopes, only changing the single-use module with its tube with the mini-camera and the working channel, maintaining the reusable module that does not come into contact with the patient, which is the grip with its electrical connections to the monitors and which may be re-sterilized if necessary.

The module that comes into contact with the patient is single-use, and the module that does not come into contact with the patient is reusable, and both modules are easily coupled together or detached.

The module which does not come into contact with the patient consists of a body which preferably opens by its longitudinal half, with a hinge system, to be able, when it is open, to couple therein the module that does come into contact with the patient. Later, on closing the cover, (the two parts of the grip), it becomes a videoscope with its form of previous grip.

The present invention, as it has an innovative modular grip, which allows separating grip and tube in separate modules, manages to cheapen the costs of these videoscopes which are enormous for hospitals.

### Brief description of the figures

The following figures have been included as part of the explanation of at least one embodiment of the invention.
Figure 1: Shows an exploded perspective view of an embodiment of the videoscope, wherein it is possible to observe the videoscope grip, the screen configured to be coupled in the grip, and the part of the single-use tube configured to be introduced in the grip.
Figure 2: Shows a perspective view, wherein the grip is in its open position, and the two parts of the grip appear articulated around the hinge.
Figure 3: Shows a side view of the grip of figures 1 and 2, in its closed position.
Figure 4: Shows a perspective view of an embodiment of the flexible tip.
Figure 5: Shows a front view of the flexible tip of Figure 4.
Figure 6: Shows a detail of the flexible tip of Figures 4 and 5, with sectional view of the three types of links.
Figure 7: Shows a view of a flexible tip which allows the movement in two perpendicular planes (4 axes), i.e. upward/downward and left/right.
Figure 8: Shows a view of the grip of a videoscope/fiberscope, with anti-pricking protector shield.
Figure 9: Shows a schematic view of a pulley system for tensioning cables with double fastening.

### Detailed description

The present invention relates, as mentioned above, to a videoscope or fiberscope.

One of the main characteristics of the videoscope/fiberscope object of the present invention is that it has a modular characteristic, which allows making the grip (100) or main body of the videoscope independent from the tube (200) with the mini-camera.

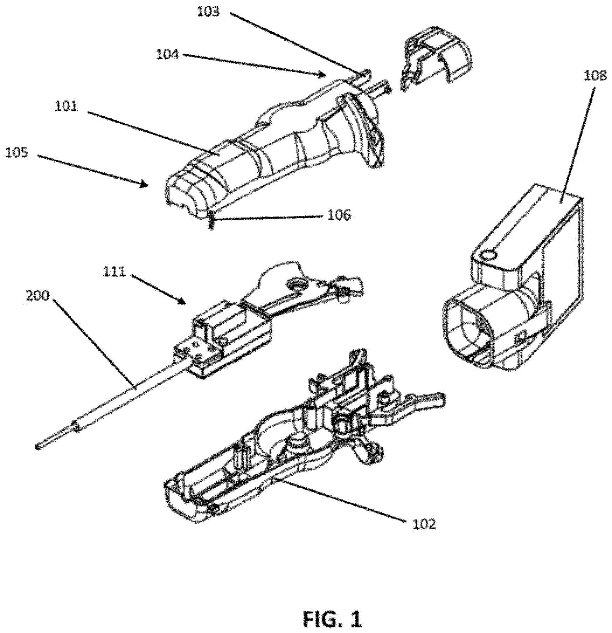

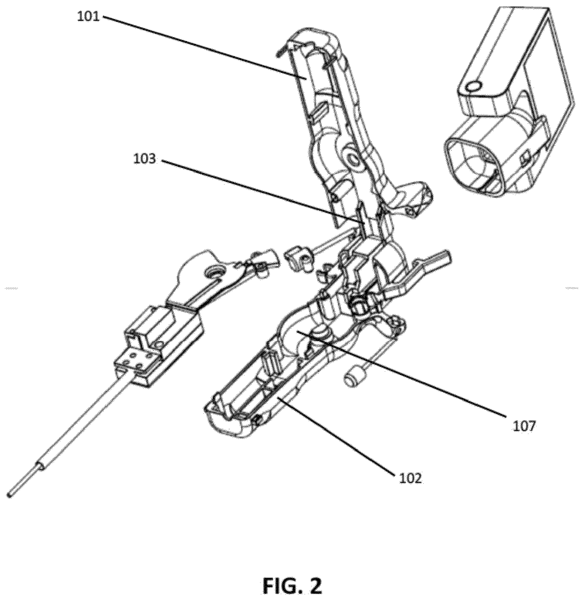

Thus, the videoscope comprises (see Figures 1 to 3) a grip (100) formed by two parts, a first part (101) and a second part (102), articulated by means of a hinge (103) present in a first end (104) of the grip (100), and with closing means (106) or clipped on a second end (105) of the grip (100).

When said grip (100) opens, by means of a relative rotation of the two parts (101, 102) in articulated form around the hinge (103), it accesses an inner cavity (107) of the grip.

Inside said cavity (107), there are mechanical connection means, electricity connection means and electronic connection means for the connection of the tube (200) with the mini-camera to said grip (100). These mechanical, electrical and electronic means are preferably made on a connection piece (111), configured to be inserted inside the cavity.

According to one possible embodiment, the electronic connection means are connected to a data outlet port, for the connection of a data cable and/or a removable data storage unit to the videoscope grip (100). According to another possible embodiment, the electronic connection means are connected to an internal data storage unit. Likewise, the electronic connection means may be connected to a screen (108) coupled to the grip (100)

According to one possible embodiment, the electricity connection means are connected to an internal battery situated in the videoscope grip (100). According to another possible embodiment, the electricity connection means are connected to a connection port of the videoscope grip (100) for the connection of a power cable configured to be connected to an external power source.

The mechanical connection means allows stabilizing the proximal end of the tube (200) in the cavity (107) of the videoscope grip (100).

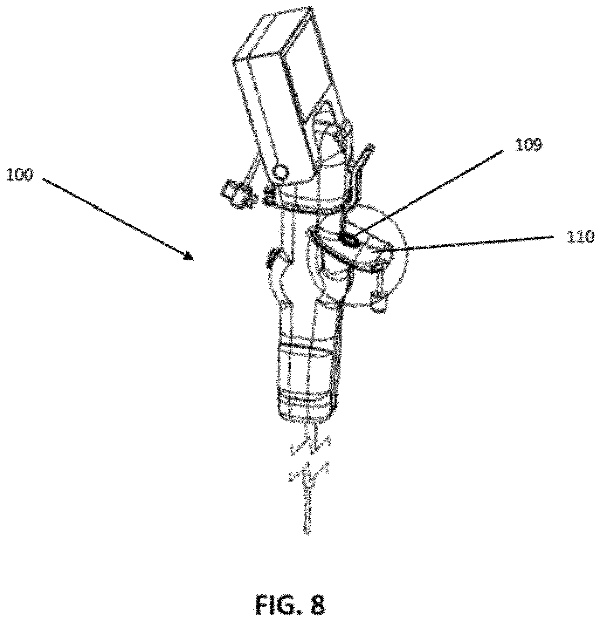

According to a preferred embodiment, the videoscope comprises a working channel with an inlet (109) for the introduction of needles, where said inlet (109) is situated in the videoscope grip (100). In this case, preferably, the videoscope/fiberscope grip (100) comprises a protector shield (110) (see Figure 8) between the inlet (109) for the introduction of needles and a gripping area of the grip (100). The gripping area is configured with surface dimensions and/or characteristics which make it suitable for the gripping thereof by the medical staff using the videoscope.

Said protector shield (110) manages to totally decrease or avoid the danger that a nursing assistant pricks with the needle the person holding or gripping the grip (100) by the gripping area, during the insertion manoeuvre of the needle through the inlet (109) of the working channel.

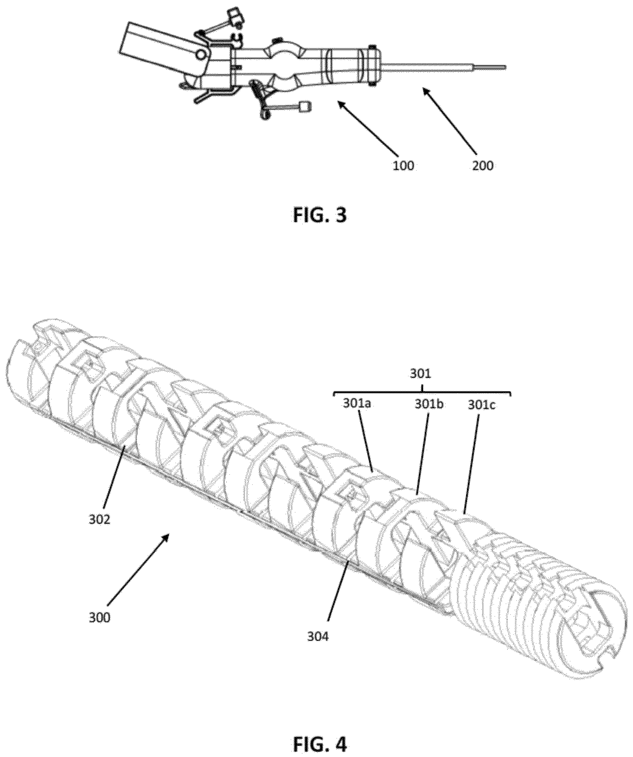

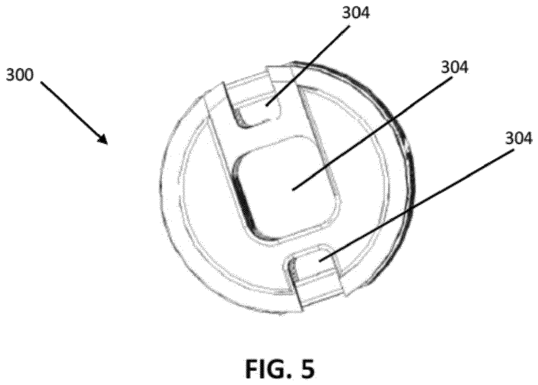

Likewise, according to a preferred embodiment (see Figures 4 to 6), the videoscope comprises a flexible tip (300) configured to allow the movement of the mini-camera in a movement plane, according to two directions (upward and downward, or left and right) i.e. configured to allow mobility of the mini-camera in two axes.

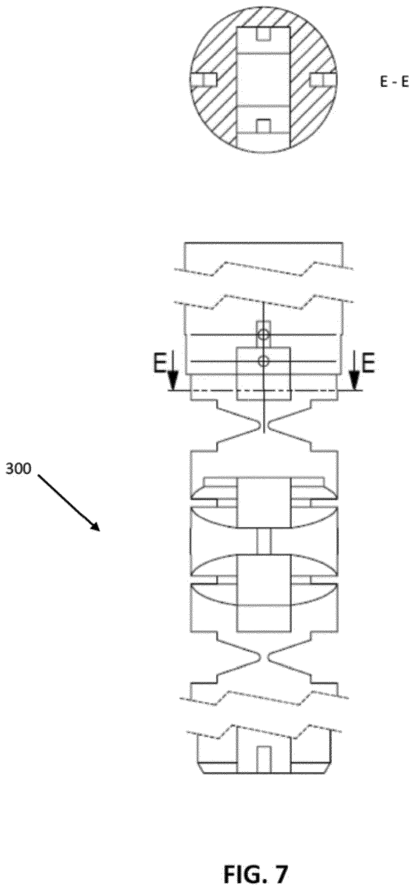

According to another possible embodiment (see Figure 7), the flexible tip (300) of the videoscope is configured to allow the movement of the mini-camera in two perpendicular planes of movement according to four directions (upward/downward and left/right), i.e. configured to allow mobility of the mini-camera in four axes.

The flexible tip (300) is preferably manufactured by means of plastic injection (typically medical plastic).

According to a preferred embodiment, the flexible tip (300) is composed of three types of units or links (301) or discs. For convenience in the description, said links (301) are differentiated below, between a first type (301a), a second type (301b) and a third type (301c) of link (301), respectively with the classification "DO", "RE" and "MI".

Each link (301) is joined to the one before and/or after by means of two columns (302) or ribs. These columns (302) have a thin geometry, with a narrowing (303) in an intermediate section between two adjacent links (301). This narrowing (303) of the plastic column (302) allows the bending/articulation, between every two links (301), of the flexible tip (300), without the need to have mechanical shafts and articulations between different pieces, as occurs in the articulated tips of conventional videoscopes.

The links (301) or discs have a geometry with curves or hollows.

On disposing the three types of links (301) "DO", "RE", "MI" suitably together with one another, independent channels (304), observables in the axial direction of the flexible tip (300), are created. These independent channels allow introducing by different routes the tensioning cables (305) or movement cables and the electric/electronic wiring for the transmission of power and image data.

According to a preferred embodiment of the flexible tip (300), there are at least three channels (304): a central channel, of greatest section, configured for the introduction of the electrical/electronic connections, and two side channels (left/right or top/bottom), configured for the introduction, in each one of them, of the corresponding tensioning cable (305) or movement cable.

According to the embodiment (see Figure 7) of the flexible tip (300) for rotations in two planes, there are at least five channels (304): a central channel, of greatest section, configured for the introduction of the electrical/electronic connections, two side or peripheral channels one on the right and one the left, and two side or peripheral channels (one top and another bottom), configured for the introduction, in each one of them, of the corresponding tensioning cable (305) or movement cable for each one of the four possible rotations.

There are different possible groupings of the links (301) or discs.

According to a possible grouping, the links may be distributed in groups of three identical links (301), preceded and/or followed by groups of three different links (301), i.e.: "DO-DO-DO-RE-RE-RE-MI-MI-MI-DO-DO-DO...".

According to another possible embodiment (see Figure 4), each link (301) is preceded and/ or followed by another link (301) of different type, i.e.: "DO-RE-MI-DO-RE-MI-DO...".

According to another possible embodiment, the links (301) are grouped in groups of two identical links (301), preceded and/or followed by groups of two different links (301), i.e.: "DO-DO-RE-RE-MI-MI-DO-DO...".

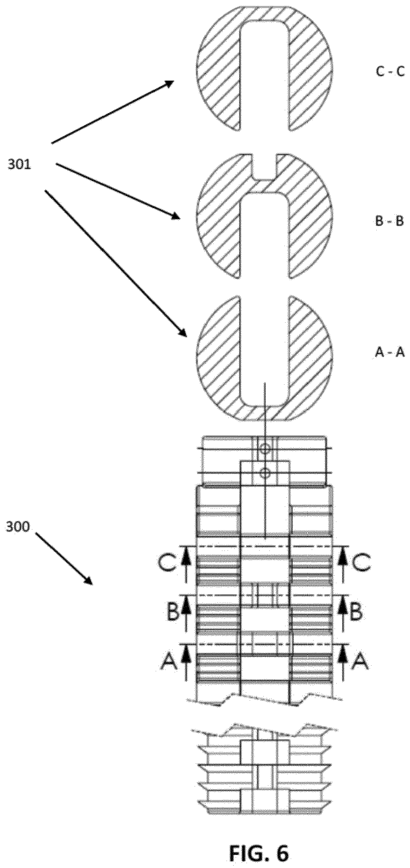

Typically (see Figure 6), two of the different types of links (301) or discs are in fact the same type of disc, although placed inverted.

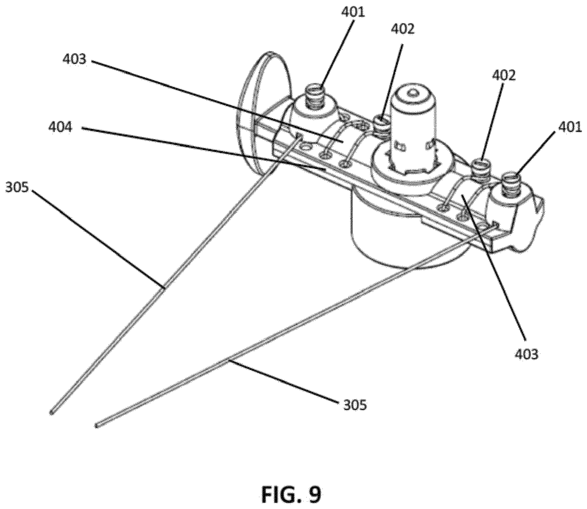

Preferably, in conjunction with the previous characteristics, or independently from them, the videoscope has a joining system (see Figure 9) between the tensioning cables (305) and the handle or actuator, which is composed of two fastenings (401, 402) and a pulley (403) situated on a support (404). The first fastening (401) is for the pretensioning of the tensioning cables (305) in the assembly of the videoscope in the production chain; this pretensioning does not serve to withstand the force exerted on the handle, but only for the correct initial tensioning of the tensioning cables (305); after the first fastening (305) the pulley (403) is positioned, which is used to withstand the actuation with force of the handle; finally, after the pulley (403), the second fastening (402) is positioned, which serves to fasten the distal end of the tensioning cable (305) after exiting the pulley (403), which it fastens so that the tensioning cable (305) which is wound round the pulley (403) does not come off. This double fastening system (401, 402) with pulley (403) allows achieving, with the first fastening (401), an optimum initial tensioning of the tensioning cables (305) in the device's production chain; later, with the pulley (403) which allows withstanding the greatest force exerted by the doctor on the handle during its use; and the second fastening (402) fastens the tensioning cables (305) so that the tensioning cable does not come off the pulley (403).

## Claims

1. Videoscope comprising a grip (100) and a tube (200) equipped with a mini-camera at a distal end of the tube (200), **characterized in that** the grip (100) comprises a first part (101) and a second part (102) articulated together by means of a hinge (103) situated at a first end (104) of the grip (100), and where the grip (100) comprises closing means (106) of the two parts (101, 102), so that the grip (100) is susceptible of being disposed in an open position and a closed position, where the grip (100) comprises an inner cavity (107) accessible in the open position of the grip (100), and where the cavity (107) of the grip (100) is configured to house a connection piece (111) comprising: mechanical connection means with a proximal end of the mini-camera tube (200), configured to stabilize the proximal end of the tube (200) in the cavity (107) of the grip (200); electric power connection means, configured to transmit electrici power to the mini-camera, and; electronic connection means with the mini-camera tube (200), configured for image data transmission.

2. Videoscope according to claim 1, **characterized in that** the electronic connection means are connected to a data outlet port, for the connection of a data cable and/or a removable data storage unit to the videoscope grip (100).

3. Videoscope comprising a grip (100) and a tube (200) equipped with a mini-camera at a distal end of the tube (200), where the tube comprises a working channel configured for the passage of a needle or biopsy means through the inside of said tube, where the grip comprises an inlet (109) of the working channel, said inlet (109) being configured for the introduction of the needle or the biopsy means, **characterized in that** the grip (100) comprises a protector shield (110) between said inlet (109) and a gripping area of the grip (100).

4. Flexible tip (300) for videoscope, **characterized in that** it is made in a single piece and comprises a plurality of links (301) joined together by means of at least one column (302), where the at least one column (302) comprises a narrowing (303) in a central section between every two links (301), thus allowing the bending of the flexible tip (300) in each area of narrowing (303) of the column (302), where each link (301) comprises a geometry with a plurality of curves or hollows such that, on disposing links (301) adjacent to other links (301), said curves or hollows generate axial channels (304) of the flexible tip (300), configured for the separate introduction of tensioning cables (305) for movement of the flexible tip (300) and of electric and electronic cables for power supply of the mini-camera and for image data transmission.

5. Flexible tip (300) according to claim 4, **characterized in that** it comprises at least three channels (304): a central channel configured for the electric and electronic cables, and two side channels configured for the introduction of the tensioning cables, where the side channels are of smaller section than the central channel.

6. Flexible tip (300) according to any of claims 4 or 5, **characterized in that** it comprises at least two columns (302).

7. Flexible tip (300) according to any of claims 4 to 6, **characterized in that** it is manufactured by means of plastic injection.

8. Flexible tip (300) according to any of claims 4 to 7, **characterized in that** it comprises three different types (301a, 301b, 301c) of links (301).

9. Flexible tip (300) according to claim 8, **characterized in that** the links (301) are grouped so that each link (301) of a determined type is preceded and/or followed by a link (301) of a different type.

10. Flexible tip (300) according to claim 8, **characterized in that** the links (301) are grouped in threes, forming groups of three links (301) of the same type and where each group is preceded and/or followed by a group of links (301) of a different type.

11. Flexible tip (300) according to claim 8, **characterized in that** the links (301) are grouped in twos, forming groups of two links (301) of the same type and where each group is preceded and/or followed by a group of links (301) of a different type.

12. Videoscope comprising a grip (100) and a tube (200) equipped with a mini-camera at a distal end of the tube (200), **characterized in that** the distal end of the tube (200) comprises the flexible tip (300) according to any of claims 4 to 11.

13. Joining system between a handle and a tensioning cable (305) for videoscope, **characterized in that** it comprises:
∘ a pulley (403), situated on a support (404), where the pulley (403) is configured for the winding of the tensioning cable (305), and to withstand a traction force exerted by the handle;
∘ a first fastening (401), situated in correspondence with a first end area of the pulley (403), where said first fastening (401) is configured to pretension the tensioning cable (305), and;
∘ a second fastening (402), situated in correspondence with a second end area of the pulley (403), where said second fastening (402) is configured to fasten the tensioning cable (305) and avoid that said tensioning cable (305) comes off the pulley (403).

14. Joining system according to claim 13, **characterized in that** it comprises:
∘ two pulleys (403) situated on the support (404);
∘ two first fastenings (401), situated respectively in correspondence with the first end area of each pulley (403), and;
∘ two second fastenings (402), situated respectively in correspondence with the second end area of each pulley (403).

15. Videoscope comprising a grip (100) and a tube (200) equipped with a mini-camera at a distal end of the tube (200), where it is configured to allow a movement of the mini-camera by means of a handle and at least one tensioning cable (305) which runs through the inside of the tube (200), **characterized in that** the videoscope comprises a joining system between the handle and the at least one tensioning cable according to any of claims 13 or 14.
